# EUROPEAN PATENT APPLICATION

(11) **EP 4 613 766 A1**
(43) Date of publication of application: **10.09.2025**
(21) Application number: 23884932.7
(22) Date of filing: 31.10.2023
(51) Int. Cl.: C07K 16/28, C12N 1/19, C12N 1/21, A61K 39/00, A61P 35/00

(54) **ANTI-CCR8 ANTIBODY AND USE THEREOF**

(30) Priority: 04.11.2022 CN 202211377345
(71) Applicant: Biotheus Inc., Tangjiawan Town, Xiangzhou District Zhuhai, Guangdong 519080 (CN)
(72) Inventor: DAI, Shuang, Zhuhai, Guangdong 519080 (CN); ZHAI, Tianhang, Zhuhai, Guangdong 519080 (CN); HUANG, Weifeng, Zhuhai, Guangdong 519080 (CN); TSUN, Andy, Zhuhai, Guangdong 519080 (CN)
(74) Representative: Ghirardi, Valeria
(86) International application number: PCT/CN2023/128490
(87) International publication number: WO 2024/094003

(57) **Abstract**

The present application relates to the field of biopharmaceuticals, and specifically relates to an antibody specifically binding to CCR8 or an antigen-binding fragment thereof, and a pharmaceutical composition and a kit comprising the same. The antibody or antigen-binding fragment thereof has ADCC activity and/or ADCP activity, and at the same time has a very good tumor inhibitory effect. Therefore, the present invention further relates to use of the antibody or the antigen-binding fragment thereof in the prevention and/or treatment of a tumor.

## Description

### Technical Field

The present application relates to the field of biomedicine, and specifically, the present application relates to an antibody or antigen-binding fragment thereof capable of specifically binding to CCR8, and to a pharmaceutical composition and a kit comprising the antibody or antigen-binding fragment thereof. The antibody or antigen-binding fragment thereof has ADCC activity and/or ADCP activity and has a good tumor inhibitory effect. Therefore, the present invention further relates to the use of the antibody or antigen-binding fragment thereof in the prevention and/or treatment of tumors.

### Background Art

In recent years, the tumor immunotherapy represented by anti-PD-1/PD-L1 monoclonal antibodies has made breakthrough progress. However, the overall response rate of this therapy in solid tumors is only 10% to 30% (Ribas A, Wolchok JD. Cancer immunotherapy using checkpoint blockade. Science, 2018, 359(6382): 1350-1355). Therefore, the development of new immunotherapies to improve the response rate and survival of tumor patients remains an unmet clinical need.

Several studies have shown that there are large numbers of regulatory T cells (Treg) infiltrating in the tumor microenvironment, which inhibit the immune response through mechanisms such as competitive binding to CD80 and CD86 activation signals by cytotoxic T lymphocyte-associated antigen-4 (CTLA-4) highly expressed on the cell surface, competitive binding to IL-2 by the high-affinity IL-2 receptor CD25, and release of immunosuppressive factors such as TGF-β and IL-10, thereby promoting tumor occurrence and development (Setoguchi R, Hori S, Takahashi T, et al. Homeostatic maintenance of natural Foxp3(+) CD25(+) CD4(+) regulatory T cells by interleukin (IL)-2 and induction of autoimmune disease by IL-2 neutralization. J Exp Med, 2005, 201(5): 723-735). Therefore, many pharmaceutical companies and research institutions are committed to developing therapies to deplete Tregs in the tumor microenvironment, hoping to relieve immunosuppression and increase the patient's immune response to tumors. Among these, several monoclonal antibodies that separately target CD25, CCR4 and CTLA-4 highly expressed by Tregs and exhibit antibody-dependent cell-mediated cytotoxicity (ADCC) have entered the clinical research. However, these antibodies have limited response rates in the treatment of solid tumors and face potential side effects caused by the depletion of peripheral Tregs.

Unlike CTLA-4, CD25, CCR4 and other immune checkpoint molecules that are highly expressed on T cells or peripheral Treg cells, chemokine (C-C motif) receptor 8 (CCR8) is specifically highly expressed on tumor-infiltrating Treg cells (Plitas G, Konopacki C, Wu K, et al. Regulatory T cells exhibit distinct features in human breast cancer. Immunity, 2016, 45(5): 1122-1134). Monoclonal antibodies targeting CCR8 have the potential to specifically eliminate tumor-infiltrating Tregs and avoid killing peripheral Tregs. Therefore, there is a need in the art to develop a novel CCR8-targeting antibody that can efficiently and specifically eliminate tumor-infiltrating Tregs, while avoid the toxic side effects of eliminating peripheral Tregs, thereby being useful in the treatment of diseases, particularly cancer.

### Contents of the present invention

The inventors of the present application have screened and obtained a monoclonal antibody against CCR8 after extensive research, and further, a series of engineering modifications were performed on the heavy chain constant region of the antibody. The obtained antibodies have ADCC and/or ADCP activity, and these antibodies have high binding affinity and good specificity to CCR8. Some of these antibodies have enhanced ADCC or ADCP activity, and some of these antibodies have enhanced ADCC and ADCP activity at the same time. In animal models, the administration of the antibodies of the present invention can significantly inhibit tumor growth. Based on this, the present application also provides a composition comprising the antibody or antigen-binding fragment thereof, a nucleic acid encoding the antibody or antigen-binding fragment thereof and a host cell containing the same, as well as related uses thereof.

Therefore, in one aspect, the present invention provides an antibody or antigen-binding fragment thereof capable of specifically binding to CCR8, wherein the antibody or antigen-binding fragment thereof comprises:
(a) a heavy chain variable region (VH) comprising the following 3 complementarity determining regions (CDRs):
   (i) a VH CDR1, which consists of the following sequence: SEQ ID NO: 1, or a sequence having a substitution, deletion or addition of one or several amino acids (e.g., a substitution, deletion or addition of 1, 2 or 3 amino acids) as compared thereto,
   (ii) a VH CDR2, which consists of the following sequence: SEQ ID NO: 2, or a sequence having a substitution, deletion or addition of one or several amino acids (e.g., a substitution, deletion or addition of 1, 2 or 3 amino acids) as compared thereto, and
   (iii) a VH CDR3, which consists of the following sequence: SEQ ID NO: 3, or a sequence having a substitution, deletion or addition of one or several amino acids (e.g., a substitution, deletion or addition of 1, 2 or 3 amino acids) as compared thereto;
      and/or,
(b) a light chain variable region (VL) comprising the following 3 complementarity determining regions (CDRs):
   (iv) a VL CDR1, which consists of the following sequence: SEQ ID NO: 5, or a sequence having a substitution, deletion or addition of one or several amino acids (e.g., a substitution, deletion or addition of 1, 2 or 3 amino acids) as compared thereto,
   (v) a VL CDR2, which consists of the following sequence: SEQ ID NO: 6, or a sequence having a substitution, deletion or addition of one or several amino acids (e.g., a substitution, deletion or addition of 1, 2 or 3 amino acids) as compared thereto, and
   (vi) a VL CDR3, which consists of the following sequence: SEQ ID NO: 7, or a sequence having a substitution, deletion or addition of one or several amino acids (e.g., a substitution, deletion or addition of 1, 2 or 3 amino acids) as compared thereto.

In certain embodiments, the substitution as described in any one of (i) to (vi) is a conservative substitution.

In certain embodiments, the CDRs described in any one of (i) to (vi) are defined according to the Kabat, IMGT or Chothia numbering systems.

In certain embodiments, the CDRs described in any one of (i) to (vi) are defined according to the IMGT numbering system.

In certain embodiments, the antibody or antigen-binding fragment thereof comprises: the following 3 heavy chain CDRs: a VH CDR1 as set forth in SEQ ID NO: 1, a VH CDR2 as set forth in SEQ ID NO: 2, a VH CDR3 as set forth in SEQ ID NO: 3; and/or, the following 3 light chain CDRs: a VL CDR1 as set forth in SEQ ID NO: 5, a VL CDR2 as set forth in SEQ ID NO: 6, a VL CDR3 as set forth in SEQ ID NO: 7.

In certain embodiments, the antibody or antigen-binding fragment thereof comprises:
(a) a heavy chain variable region (VH), which comprises an amino acid sequence selected from the group consisting of:
   (i) a sequence as set forth in SEQ ID NO: 4;
   (ii) a sequence having a substitution, deletion or addition of one or several amino acids (e.g., a substitution, deletion or addition of 1, 2, 3, 4 or 5 amino acids) as compared to the sequence as set forth in SEQ ID NO: 4; and
   (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as set forth in SEQ ID NO: 4;
      and/or
(b) a light chain variable region (VL), which comprises an amino acid sequence selected from the group consisting of:
   (iv) a sequence as set forth in SEQ ID NO: 8;
   (v) a sequence having a substitution, deletion or addition of one or several amino acids (e.g., a substitution, deletion or addition of 1, 2, 3, 4 or 5 amino acids) as compared to the sequence as set forth in SEQ ID NO: 8; and
   (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 8.

In certain embodiments, the substitution described in (ii) or (v) is a conservative substitution.

In certain embodiments, the antibody or antigen-binding fragment thereof comprises: a VH having a sequence as set forth in SEQ ID NO: 4 and a VL having a sequence as set forth in SEQ ID NO: 8.

In certain embodiments, the antibody or antigen-binding fragment thereof comprises a constant region derived from a mammalian immunoglobulin or a variant thereof.

In certain embodiments, the antibody or antigen-binding fragment thereof comprises:
(a) a heavy chain constant region (CH) of a mammalian immunoglobulin or a variant thereof, wherein the variant has a substitution, deletion or addition of one or several amino acids or any combination thereof (e.g., a substitution, deletion or addition of up to 20, up to 15, up to 10, or up to 5 amino acids or any combination thereof; e.g., a substitution, deletion or addition of 1, 2, 3, 4 or 5 amino acids or any combination thereof) as compared to the sequence from which it is derived; and/or
(b) a light chain constant region (CL) of a mammalian immunoglobulin or a variant thereof, wherein the variant has a substitution, deletion or addition of one or several amino acids or any combination thereof (e.g., a substitution, deletion or addition of up to 20, up to 15, up to 10, or up to 5 amino acids or any combination thereof; e.g., a substitution, deletion or addition of 1, 2, 3, 4 or 5 amino acids or any combination thereof) as compared to the sequence from which it is derived.

In certain embodiments, the mammal is selected from the group consisting of a mouse and a human.

In certain embodiments, the heavy chain constant region is selected from the group consisting of IgG, IgM, IgE, IgD and IgA.

In certain embodiments, the heavy chain constant region is a mouse or human IgG heavy chain constant region; for example, a human IgG1, IgG2, IgG3 or IgG4 heavy chain constant region; for example, a mouse IgG1, IgG2a, IgG2b, IgG2c or IgG3 heavy chain constant region.

In certain embodiments, the heavy chain constant region is selected from the group consisting of a human IgG1 heavy chain constant region and a mouse IgG2a heavy chain constant region.

In certain embodiments, the variant is a variant of a human IgG1 heavy chain constant region.

In certain embodiments, the variant is mutated to alanine at the position corresponding to the position 119 of the human IgG1 heavy chain constant region.

In certain embodiments, the antibody or antigen-binding fragment thereof is hypofucosylated or afucosylated.

In certain embodiments, the antibody or antigen-binding fragment thereof comprises a heavy chain constant region (CH) as set forth in SEQ ID NO: 9, 10 or 13.

In certain embodiments, the light chain constant region is a κ light chain constant region or a λ light chain constant region.

In certain embodiments, the light chain constant region is a mouse or human κ light chain constant region.

In certain embodiments, the antibody or antigen-binding fragment thereof comprises a light chain constant region (CL) as set forth in SEQ ID NO: 11 or 14.

In certain embodiments, the antigen-binding fragment is selected from the group consisting of Fab, Fab', (Fab')², Fv, disulfide-linked Fv, scFv, diabody and single-domain antibody (sdAb).

In certain embodiments, the antibody or antigen-binding fragment thereof of the present invention is a murine antibody, a chimeric antibody, a humanized antibody or a multispecific antibody.

In certain embodiments, the antibody or antigen-binding fragment thereof described in any of the above embodiments further has a feature selected from the group consisting of:
(1) having ADCC activity, such as inducing killing of a CCR8-expressing cell (e.g., a tumor cell) through ADCC; in certain embodiments, the hypofucosylated or afucosylated antibody or antigen-binding fragment thereof has stronger ADCC activity; in such embodiments, the antibody prepared by a modified host cell (e.g., a fucose-knockout CHO cell) is a hypofucosylated or afucosylated antibody;
(2) having ADCP activity, such as inducing killing of a CCR8-expressing cell (e.g., a tumor cell) through ADCP; in certain embodiments, the antibody or antigen-binding fragment thereof comprising a mutated heavy chain constant region (e.g., as set forth in SEQ ID NO: 10) has stronger ADCP activity; and/or,
(3) inhibiting tumor growth; in certain embodiments, the antibody or antigen-binding fragment thereof in combination with an additional pharmaceutically active agent (e.g., anti-PD-L1/TGF-βRII fusion protein) has a stronger ability to inhibit tumor growth.

In another aspect, the present invention provides an isolated nucleic acid molecule, which encodes the antibody or antigen-binding fragment thereof as described above.

In another aspect, the present invention provides a vector, which comprises the nucleic acid molecule as described above.

In certain embodiments, the vector of the present invention is selected from the group consisting of plasmid, cosmid, phage, and lentivirus. In certain embodiments, the vector is capable of expressing the antibody or antigen-binding fragment thereof of the present invention in a subject (e.g., a mammal, such as a human). In certain embodiments, the vector is a cloning vector or an expression vector.

In another aspect, the present invention provides a host cell, which comprises the nucleic acid molecule as described above or the vector as described above.

In certain embodiments, the host cell is a mammalian cell.

In certain embodiments, the host cell is a fucose knockout or non-knockout cell.

In such embodiments, the antibody prepared by the modified host cell (e.g., fucose knockout CHO cell) is a hypofucosylated or afucosylated antibody.

The host cell may be an eukaryotic cell (e.g., a mammalian cell, an insect cell, a yeast cell) or a prokaryotic cell (e.g., *E. coli*). Suitable eukaryotic cells include, but are not limited to, NS0 cells, Vero cells, Hela cells, COS cells, CHO cells, ExpiCHO cells, HEK293 cells, Expi293 cells, BHK cells, and MDCKII cells. Suitable insect cells include, but are not limited to, Sf9 cells. In certain embodiments, the host cell of the present invention is a mammalian cell, such as CHO (e.g., CHO-K1, CHO-S, CHO DXB11, ExpiCHO, CHO DG44).

**In** another aspect, the present invention provides a method for preparing the antibody or antigen-binding fragment thereof as described above, comprising culturing the host cell as described above under a condition that allows expression of the antibody or antigen-binding fragment thereof, and recovering the antibody or antigen-binding fragment thereof from a culture of the cultured host cell.

**In** another aspect, the present invention provides a multispecific molecule, which comprises the antibody or antigen-binding fragment thereof as described above.

**In** certain embodiments, the multispecific molecule specifically binds to CCR8 and additionally specifically binds to one or more other targets.

**In** certain embodiments, the multispecific molecule is a bispecific molecule.

In certain embodiments, the bispecific molecule further comprises a molecule having a second binding specificity for a second target (e.g., a second antibody).

In another aspect, the present invention provides an immunoconjugate, which comprises the antibody or antigen-binding fragment thereof as described above or the multispecific molecule as described above, and a therapeutic agent linked to the antibody or antigen-binding fragment thereof or the multispecific molecule.

In certain embodiments, the therapeutic agent is selected from a cytotoxic agent.

In certain embodiments, the therapeutic agent is selected from the group consisting of alkylating agent, mitotic inhibitor, antitumor antibiotic, antimetabolite, topoisomerase inhibitor, tyrosine kinase inhibitor, radionuclide agent, and any combination thereof.

In certain embodiments, the immunoconjugate is an antibody-drug conjugate (ADC).

In another aspect, the present invention provides a pharmaceutical composition, which comprises the antibody or antigen-binding fragment thereof as described above, or the multispecific molecule as described above, or the immunoconjugate as described above, and a pharmaceutically acceptable carrier and/or excipient.

In certain embodiments, the pharmaceutical composition further comprises an additional pharmaceutically active agent.

In certain embodiments, the additional pharmaceutically active agent is a drug with an anti-tumor activity, such as an alkylating agent, a mitotic inhibitor, an anti-tumor antibiotic, an antimetabolite, a topoisomerase inhibitor, a tyrosine kinase inhibitor, a radionuclide agent, a radiosensitizer, an anti-angiogenic agent, a cytokine, a molecular targeted drug, an immune checkpoint inhibitor or an oncolytic virus.

In certain embodiments, the antibody or antigen-binding fragment thereof, multispecific molecule or immunoconjugate and the additional pharmaceutically active agent are provided as separate components or as components in the same composition.

In certain embodiments, the additional pharmaceutically active agent is an anti-PD-L1/TGF-βRII fusion protein. In certain embodiments, the anti-PD-L1/TGF-βRII fusion protein has a heavy chain amino acid sequence as set forth in SEQ ID NO: 15, and a light chain amino acid sequence as set forth in SEQ ID NO: 16.

In another aspect, the present invention provides a kit, which comprises the antibody or antigen-binding fragment thereof as described above.

In some embodiments, the antibody or antigen-binding fragment thereof comprises a detectable label, such as an enzyme (e.g., horseradish peroxidase), a radionuclide, a fluorescent dye, a luminescent substance (e.g., a chemiluminescent substance), or biotin.

In some embodiments, the kit further comprises a second antibody, which is capable of specifically recognizing the antibody or antigen-binding fragment thereof as described above.

In some embodiments, the second antibody further comprises a detectable label, such as an enzyme (e.g., horseradish peroxidase), a radionuclide, a fluorescent dye, a luminescent substance (e.g., a chemiluminescent substance), or biotin.

In another aspect, the present invention provides a chimeric antigen receptor, which comprises an antigen-binding domain of the antibody or antigen-binding fragment thereof as described above.

In some embodiments, the antigen-binding domain comprises a heavy chain variable region and a light chain variable region of the antibody or antigen-binding fragment thereof as described above.

In some embodiments, the antigen-binding domain is a scFv.

In some embodiments, the chimeric antigen receptor is expressed by an immune effector cell (e.g., a T cell).

In another aspect, the present invention provides a method for inhibiting growth of a tumor cell expressing CCR8 and/or killing the tumor cell, which comprises contacting the tumor cell with an effective amount of the antibody or antigen-binding fragment thereof as described above, or the multispecific molecule as described above, or the immunoconjugate as described above, or the pharmaceutical composition as described above, or the chimeric antigen receptor as described above.

In another aspect, the present invention provides a use of the antibody or antigen-binding fragment thereof as described above, or the multispecific molecule as described above, or the immunoconjugate as described above, or the pharmaceutical composition as described above, or the chimeric antigen receptor as described above, in the manufacture of a medicament for preventing and/or treating a tumor in a subject (e.g., a human).

In certain embodiments, the medicament further comprises an additional pharmaceutically active agent.

In certain embodiments, the additional pharmaceutically active agent is a drug having an anti-tumor activity, such as an alkylating agent, a mitotic inhibitor, an anti-tumor antibiotic, an antimetabolite, a topoisomerase inhibitor, a tyrosine kinase inhibitor, a radionuclide agent, a radiosensitizer, an anti-angiogenic agent, a cytokine, a molecular targeted drug, an immune checkpoint inhibitor, or an oncolytic virus.

In certain embodiments, the additional pharmaceutically active agent is an anti-PD-L1/TGF-βRII fusion protein. In certain embodiments, the anti-PD-L1/TGF-βRII fusion protein has a heavy chain amino acid sequence as set forth in SEQ ID NO: 15, and a light chain amino acid sequence as set forth in SEQ ID NO: 16.

In certain embodiments, the tumor expresses CCR8.

In certain embodiments, the tumor involves a tumor cell expressing CCR8. In certain embodiments, the CCR8 is expressed on the surface of the tumor cell.

In certain embodiments, the tumor is selected from the group consisting of non-small cell lung cancer, small cell lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoids, Merkel cell carcinoma and other hematological malignancies, such as classical Hodgkin lymphoma (CHL), primary mediastinal large B-cell lymphoma, T-cell/histiocyte-rich B-cell lymphoma, EBV-positive and negative PTLD and EBV-associated diffuse large B-cell lymphoma (DLBCL), plasmablastic lymphoma, extranodal NK/T-cell lymphoma, nasopharyngeal carcinoma and HHV8-associated primary effusion lymphoma, Hodgkin lymphoma, central nervous system (CNS) tumors, such as primary CNS lymphoma, spinal axis tumor, brainstem glioma.

In some embodiments, the subject is a mammal, such as a human.

In another aspect, the present invention provides a use of the above-mentioned antibody or antigen-binding fragment thereof in the manufacture of a kit for determining whether a tumor can be treated by a CCR8-targeting anti-tumor therapy;
(1) contacting a sample containing a cell of the tumor with the antibody or antigen-binding fragment thereof as described above;
(2) detecting formation of a complex comprising the antibody or antigen-binding fragment thereof and CCR8.

In some embodiments, the antibody or antigen-binding fragment thereof comprises a detectable label.

In some embodiments, the CCR8 is a CCR8 of a mammal (e.g., a human, a mouse).

In certain embodiments, the tumor is selected from the group consisting of non-small cell lung cancer, small cell lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoids, Merkel cell carcinoma, and other hematological malignancies, such as classical Hodgkin lymphoma (CHL), primary mediastinal large B-cell lymphoma, T-cell/histiocyte-rich B-cell lymphoma, EBV-positive and negative PTLD and EBV-associated diffuse large B-cell lymphoma (DLBCL), plasmablastic lymphoma, extranodal NK/T-cell lymphoma, nasopharyngeal carcinoma and HHV8-associated primary effusion lymphoma, Hodgkin lymphoma, central nervous system (CNS) tumors, such as primary CNS lymphoma, spinal axis tumor, brainstem glioma.

### Definition of terms

In the present invention, unless otherwise specified, the scientific and technical terms used herein have the meanings commonly understood by those skilled in the art. In addition, the molecular genetics, nucleic acid chemistry, chemistry, molecular biology, biochemistry, cell culture, microbiology, cell biology, genomics and recombinant DNA operation steps used herein are conventional steps widely used in the corresponding fields. At the same time, in order to better understand the present invention, the definitions and explanations of relevant terms are provided below.

As used herein, the term "antibody" refers to an immunoglobulin molecule that is generally composed of two pairs of polypeptide chains, each pair having a light chain (LC) and a heavy chain (HC). Antibody light chains can be classified as κ and λ light chains. Heavy chains can be classified as µ, δ, γ, α or ε, and antibody isotypes are defined as IgM, IgD, IgG, IgA and IgE, respectively. In the light chain and heavy chain, a variable region and a constant region are connected by a "J" region of about 12 or more amino acids, and the heavy chain also contains a "D" region of about 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain CL. The constant domains do not directly participate in the binding of antibody to antigen, but exhibit a variety of effector functions, such as mediating the binding of immunoglobulin to host tissues or factors, including various cells of the immune system (e.g., effector cells) and the first component (C1q) of the classical complement system. The VH and VL regions can also be subdivided into regions with high variability (called complementarity determining regions (CDRs)), interspersed with more conservative regions called framework regions (FRs). Each VH and VL consists of three CDRs and four FRs arranged from the amino terminal to the carboxyl terminal in the following order: FR1, CDR1, FR2, CDR2, FR3, CDR3, FR4. The variable regions (VH and VL) of each heavy chain/light chain pair form antigen binding sites, respectively. The distribution of amino acids in each region or domain can follow the definition of Kabat, Sequences of Proteins of Immunological Interest (National Institutes of Health, Bethesda, Md. (1987 and 1991)), or Chothia & Lesk (1987) J. Mol. Biol. 196:901-917; Chothia et al. (1989) Nature 342:878-883.

As used herein, the term "complementarity determining region" or "CDR" refers to the amino acid residues in antibody variable region that are responsible for antigen binding. There are three CDRs in each of the variable regions of heavy and light chains, designated as CDR1, CDR2, and CDR3. The precise boundaries of these CDRs can be defined according to various numbering systems known in the art, such as the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al., Dev. Comparat. Immunol. 27:55-77, 2003). For a given antibody, a person skilled in the art will readily identify the CDRs defined by each numbering system. Moreover, the correspondence between different numbering systems is well known to those skilled in the art (for example, see Lefranc et al., Dev. Comparat. Immunol. 27:55-77, 2003).

In the present invention, the CDRs contained in the antibody or antigen-binding fragment thereof of the present invention can be determined according to various numbering systems known in the art. In certain embodiments, the CDRs contained in the antibody or antigen-binding fragment thereof of the present invention are preferably determined by the Kabat, Chothia or IMGT numbering systems.

As used herein, the term "framework region" or "FR" residues refers to those amino acid residues in antibody variable region other than the CDR residues defined above.

The term "antibody" is not limited to any particular method for producing the antibody. For example, it includes recombinant antibody, monoclonal antibody and polyclonal antibody. The antibody can be an antibody of different isotypes, for example, an IgG (e.g., IgG1, IgG2, IgG3 or IgG4 subtype), an IgA1, an IgA2, an IgD, an IgE or an IgM antibody.

As used herein, the terms "monoclonal antibody", "McAb" and "mAb" have the same meaning and are used interchangeably, referring to an antibody or an antibody fragment from a group of highly homologous antibody molecules, that is, a group of identical antibody molecules except for possible spontaneous natural mutations. Monoclonal antibodies have high specificity for a single epitope on an antigen. Polyclonal antibodies, as opposed to monoclonal antibodies, generally contain at least two or more different antibodies, and these different antibodies generally recognize different epitopes on an antigen. In addition, the modifier "monoclonal" only indicates that the antibody is characterized by being obtained from a highly homologous antibody group, and is not to be understood that the antibody needs to be prepared by any particular method.

The monoclonal antibodies of the present invention can be prepared by a variety of techniques, such as hybridoma technology (see, for example, Kohler et al., Nature, 256:495, 1975), recombinant DNA technology (see, for example, U.S. Patent Application 4,816,567), or phage antibody library technology (see, for example, Clackson et al. Nature 352: 624-628, 1991, or Marks et al. J. Mol. Biol. 222: 581-597, 1991).

As used herein, the term "antigen-binding fragment" of antibody refers to a polypeptide comprising a fragment of full-length antibody, which retains the ability to specifically bind to the same antigen bound by the full-length antibody, and/or competes with the full-length antibody for specific binding to the antigen, which is also referred to as an "antigen-binding portion". See generally, Fundamental Immunology, Ch. 7 (Paul, W., ed., 2nd edition, Raven Press, N.Y. (1989), which is incorporated herein by reference in its entirety for all purposes. Antigen-binding fragments of antibodies can be produced by recombinant DNA techniques or by enzymatic or chemical cleavage of intact antibodies. Non-limiting examples of antigen-binding fragments include Fab, Fab', F(ab')2, Fd, Fv, complementary determining region (CDR) fragment, scFv, diabody, single domain antibody, chimeric antibody, linear antibody, nanobody (technology from Domantis), probody, and polypeptide that contains at least a portion of antibody sufficient to confer specific antigen binding ability to the polypeptide. Engineered antibody variants are reviewed in Holliger et al., 2005; Nat Biotechnol, 23: 1126-1136.

As used herein, the term "full-length antibody" refers to an antibody consisting of two "full-length heavy chains" and two "full-length light chains". Wherein, "full-length heavy chain" refers to a polypeptide chain that is composed of a heavy chain variable region (VH), a heavy chain constant region CH1 domain, a hinge region (HR), a heavy chain constant region CH2 domain, and a heavy chain constant region CH3 domain in the direction from N-terminal to C-terminal; and, when the full-length antibody is an IgE isotype, it optionally also comprises a heavy chain constant region CH4 domain. Preferably, a "full-length heavy chain" is a polypeptide chain consisting of VH, CH1, HR, CH2 and CH3 in the direction from N-terminal to C-terminal. A "full-length light chain" is a polypeptide chain consisting of a light chain variable region (VL) and a light chain constant region (CL) in the N-terminal to C-terminal direction. Two pairs of full-length antibody chains are linked together by disulfide bonds between CL and CH1 and between the HRs of the two full-length heavy chains. The full-length antibody of the present invention may be from a single species, such as humans; and may also be a chimeric antibody or humanized antibody. The full-length antibody of the present invention comprises two antigen binding sites formed by VH and VL pairs, respectively, which specifically recognize/bind to the same antigen.

As used herein, the term "Fd" refers to an antibody fragment consisting of VH and CH1 domains; the term "dAb fragment" refers to an antibody fragment consisting of VH domain (Ward et al., Nature 341:544 546 (1989)); the term "Fab fragment" refers to an antibody fragment consisting of VL, VH, CL and CH1 domains; the term "F(ab')² fragment" refers to an antibody fragment comprising two Fab fragments linked by disulfide bridges at the hinge region; the term "Fab' fragment" refers to a fragment obtained after reduction of the disulfide bonds linking the two heavy chain fragments in the F(ab')² fragment, consisting of a complete light chain and the Fd fragment of the heavy chain (consisting of the VH and CH1 domains).

As used herein, the term "Fv" refers to an antibody fragment consisting of the VL and VH domains of a single arm of antibody. The Fv fragment is generally considered to be the smallest antibody fragment that can form a complete antigen binding site. It is generally believed that the six CDRs confer antigen binding specificity to an antibody. However, even a single variable region (e.g., Fd fragment, which contains only three CDRs specific for an antigen) can recognize and bind to the antigen, although its affinity may be lower than that of the complete binding site.

As used herein, the term "Fc" refers to an antibody fragment formed by disulfide bonding of the second and third constant regions of first heavy chain of an antibody to the second and third constant regions of second heavy chain. The Fc fragment of antibody has a variety of different functions but does not participate in antigen binding.

As used herein, the term "scFv" refers to a single polypeptide chain comprising VL and VH domains, wherein the VL and VH are connected by a linker (see, for example, Bird et al., Science 242:423-426 (1988); Huston et al., Proc. Natl. Acad. Sci. USA 85:5879-5883 (1988); and Pluckthun, The Pharmacology of Monoclonal Antibodies, Vol. 113, Roseburg and Moore, eds., Springer-Verlag, New York, pp. 269-315 (1994)). Such scFv molecules may have the general structure: NH₂-VL-linker-VH-COOH or NH₂-VH-linker-VL-COOH. A suitable prior art linker consists of repeated GGGGS amino acid sequences or variants thereof. For example, a linker having the amino acid sequence (GGGGS)₄ may be used, but variants thereof may also be used (Holliger et al. (1993), Proc. Natl. Acad. Sci. USA 90: 6444-6448). Other linkers that can be used in the present invention are described by Alfthan et al. (1995), Protein Eng. 8:725-731, Choi et al. (2001), Eur. J. Immunol. 31: 94-106, Hu et al. (1996), Cancer Res. 56:3055-3061, Kipriyanov et al. (1999), J. Mol. Biol. 293:41-56 and Roovers et al. (2001), Cancer Immunol. In some cases, a disulfide bond may also exist between the VH and VL of scFv. In certain embodiments of the present invention, the scFv may form a di-scFv, which refers to an antibody formed by connecting two or more single scFvs in series. In certain embodiments of the present invention, the scFv may form a (scFv)₂, which refers to an antibody formed by connecting two or more single scFvs in parallel.

As used herein, the term "single-domain antibody (sdAb)" has the meaning generally understood by those skilled in the art, which refers to an antibody fragment composed of a single monomeric variable antibody domain (e.g., a single heavy chain variable region) that retains the ability to specifically bind to the same antigen bound by full-length antibody. The single-domain antibody is also called nanobody.

Each of the above antibody fragments retains the ability to specifically bind to the same antigen bound by full-length antibody, and/or competes with the full-length antibody for specific binding to the antigen.

Antigen-binding fragments of antibodies (e.g., the above antibody fragments) can be obtained from a given antibody (e.g., the antibody provided by the present invention) using conventional techniques known to those skilled in the art (e.g., recombinant DNA technology or enzymatic or chemical cleavage methods), and the antigen-binding fragments of antibodies can be screened for specificity in the same manner as for intact antibodies.

As used herein, unless the context clearly indicates otherwise, when referring to the term "antibody", it includes not only intact antibody, but also an antigen-binding fragment of the antibody.

As used herein, the term "chimeric antibody" refers to an antibody in which a portion of the light chain or/and heavy chain is derived from one antibody (which may be derived from a particular species or belong to a particular antibody class or subclass), and another portion of the light chain or/and heavy chain is derived from another antibody (which may be derived from the same or different species or belong to the same or different antibody class or subclass), but in any case, it still retains binding activity to the target antigen (U.S.P 4,816,567 to Cabilly et al.; Morrison et al., Proc. Natl. Acad. Sci. USA, 81:6851 6855 (1984)). In certain embodiments, the term "chimeric antibody" may include an antibody in which the heavy chain and light chain variable regions of the antibody are derived from a first antibody, and the heavy chain and light chain constant regions of the antibody are derived from a second antibody.

As used herein, the term "identity" is used to refer to the matching of sequences between two polypeptides or between two nucleic acids. To determine the percent identity of two amino acid sequences or two nucleic acid sequences, the sequences are aligned for optimal comparison purposes (e.g., a gap may be introduced in a first amino acid sequence or nucleic acid sequence for optimal alignment with a second amino acid or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, the molecules are identical at that position. The percent identity between two sequences is a function of the number of identical positions shared by the sequences (i.e., percent identity = number of identical overlapping positions/total number of positions × 100%). In certain embodiments, the two sequences are of the same length.

The determination of the percent identity between two sequences can also be accomplished using a mathematical algorithm. A non-limiting example of mathematical algorithm for the comparison of two sequences is the algorithm of Karlin and Altschul, 1990, Proc. Natl. Acad. Sci. U.S.A. 87:2264-2268, as modified in Karlin and Altschul, 1993, Proc. Natl. Acad. Sci. U.S.A. 90:5873-5877. Such an algorithm is incorporated into the NBLAST and XBLAST programs of Altschul et al., 1990, J. Mol. Biol. 215:403.

As used herein, the term "variant", in the context of polypeptide (including polypeptide), also refers to a polypeptide or peptide comprising an amino acid sequence that has been altered by the introduction of a substitution, deletion or addition of amino acid residues. In some cases, the term "variant" also refers to a polypeptide or peptide that has been modified (i.e., by covalently attaching any type of molecule to the polypeptide or peptide). For example, but not limiting, the polypeptide can be modified, for example, by glycosylation, acetylation, pegylation, phosphorylation, amidation, derivatization by known protecting/blocking groups, proteolytic cleavage, attachment to cellular ligands or other proteins, etc. Derivatized polypeptides or peptides can be produced by chemical modification using techniques known to those skilled in the art, including but not limited to specific chemical cleavage, acetylation, formylation, metabolic synthesis of tunicamycin, etc. In addition, variants have similar, identical or improved functions to the polypeptide or peptide from which they are derived.

As used herein, the term "specific binding" or "specifically binding" refers to a non-random binding reaction between two molecules, such as a reaction between an antibody and an antigen to which it is directed. The strength or affinity of a specific binding interaction can be expressed by the equilibrium dissociation constant (KD) of the interaction. In the present invention, the term "KD" refers to the dissociation equilibrium constant of a specific antibody-antigen interaction, which is used to describe the binding affinity between an antibody and an antigen. The smaller the equilibrium dissociation constant, the tighter the antibody-antigen binding and the higher the affinity between the antibody and the antigen.

As used herein, the detectable label of the present invention can be any substance detectable by fluorescent, spectroscopic, photochemical, biochemical, immunological, electrical, optical or chemical means. Such labels are well known in the art, and examples thereof include, but are not limited to, enzymes (e.g., horseradish peroxidase, alkaline phosphatase, β-galactosidase, urease, glucose oxidase, etc.), radionuclides (e.g., ³H, ¹²⁵I, ³⁵S, ¹⁴C or ³²P), fluorescent dyes (e.g., fluorescein isothiocyanate (FITC), fluorescein, tetramethylrhodamine isothiocyanate (TRITC), phycoerythrin (PE), Texas Red, rhodamine, quantum dots or cyanine dye derivatives (e.g., Cy7, Alexa 750)), luminescent substances (e.g., chemiluminescent substances, such as acridinium ester compounds, luminol and derivatives thereof, ruthenium derivatives such as terpyridine ruthenium), magnetic beads (e.g., Dynabeads^{®}), calorimetric labels such as colloidal gold or colored glass or plastic (e.g., polystyrene, polypropylene, latex, etc.) beads, and biotin for binding to avidin (e.g., streptavidin) modified with the above labels.

As used herein, the term "vector" refers to a nucleic acid vehicle into which a polynucleotide can be inserted. When a vector can express the protein encoded by the inserted polynucleotide, the vector is called an expression vector. The vector can be introduced into a host cell by transformation, transduction or transfection so that the genetic material elements it carries are expressed in the host cell. Vectors are well known to those skilled in the art, and include but are not limited to: plasmid; phagemid; cosmid; artificial chromosome, such as yeast artificial chromosome (YAC), bacterial artificial chromosome (BAC) or P1-derived artificial chromosome (PAC); bacteriophage such as λ phage or M13 phage and animal virus. Animal viruses that can be used as vectors include but are not limited to retrovirus (including lentivirus), adenovirus, adeno-associated virus, herpes virus (e.g., herpes simplex virus), poxvirus, baculovirus, papillomavirus, papovavirus (e.g., SV40). A vector can contain a variety of elements that control expression, including but not limited to promoter sequence, transcription start sequence, enhancer sequence, selection element and reporter gene. In addition, the vector may also contain a replication origin.

As used herein, the term "host cell" refers to a cell that can be used to introduce a vector, including but not limited to a prokaryotic cell such *as Escherichia coli* or *Bacillus subtilis,* a fungal cell such as yeast cell or *Aspergillus,* an insect cell such as *S2 Drosophila* cell or Sf9, or an animal cell such as fibroblast, CHO cell, COS cell, NSO cell, HeLa cell, BHK cell, HEK 293 cell or human cell.

As used herein, the term "conservative substitution" refers to an amino acid substitution that does not adversely affect or alter the expected properties of the protein/polypeptide comprising the amino acid sequence. For example, a conservative substitution can be introduced by standard techniques known in the art, such as site-directed mutagenesis and PCR-mediated mutagenesis. Conservative amino acid substitutions include substitutions that replace amino acid residues with amino acid residues having similar side chains, such as substitutions with residues that are physically or functionally similar to the corresponding amino acid residue (e.g., having similar size, shape, charge, chemical properties, including the ability to form covalent bond or hydrogen bond, etc.). Families of amino acid residues with similar side chains have been defined in the art. These families include amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), β-branched side chains (e.g., threonine, valine, isoleucine), and aromatic side chains (e.g., tyrosine, phenylalanine, tryptophan, histidine). Thus, it is preferred to replace a corresponding amino acid residue with another amino acid residue from the same side chain family. Methods for identifying conservative substitutions of amino acids are well known in the art (see, for example, Brummell et al., Biochem. 32:1180-1187 (1993); Kobayashi et al. Protein Eng. 12(10):879-884 (1999); and Burks et al. Proc. Natl Acad. Set USA 94:412-417 (1997), which are incorporated herein by reference).

The twenty conventional amino acids referred to herein are written in accordance with conventional usage. See, for example, Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. And in the present invention, amino acids are generally represented by single-letter and three-letter abbreviations known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient, which is well known in the art (see, for example, Remington's Pharmaceutical Sciences. Edited by Gennaro AR, 19th ed. Pennsylvania: Mack Publishing Company, 1995), and includes, but is not limited to: pH regulator, surfactant, adjuvant, ionic strength enhancer, diluent, agent for maintaining osmotic pressure, agent for delaying absorption, preservative. For example, the pH regulator includes, but is not limited to, phosphate buffer. The surfactant includes, but is not limited to, cationic, anionic or nonionic surfactant, such as Tween-80. The ionic strength enhancer includes, but is not limited to, sodium chloride. The preservative includes, but is not limited to, various antibacterial and antifungal agents, such as paraben, chlorobutanol, phenol, sorbic acid, and the like. The agent for maintaining osmotic pressure includes, but is not limited to, sugar, NaCl, and the like. The agent for delaying absorption includes, but is not limited to, monostearate and gelatin. The diluent includes, but is not limited to, water, aqueous buffer (e.g., buffered saline), alcohol and polyol (e.g., glycerol), etc. The preservative includes, but is not limited to, various antibacterial and antifungal agents, such as thimerosal, 2-phenoxyethanol, paraben, chlorobutanol, phenol, sorbic acid, etc. The stabilizer has the meanings generally understood by those skilled in the art, which can stabilize the desired activity of the active ingredient in the drug, including but not limited to sodium glutamate, gelatin, SPGA, sugar (e.g., sorbitol, mannitol, starch, sucrose, lactose, dextran, or glucose), amino acid (e.g., glutamic acid, glycine), protein (e.g., dried whey, albumin or casein) or degradation product thereof (e.g., lactalbumin hydrolysate), etc. In certain exemplary embodiments, the pharmaceutically acceptable carrier or excipient includes a sterile injectable liquid (e.g., an aqueous or non-aqueous suspension or solution). In certain exemplary embodiments, such sterile injectable liquid is selected from the group consisting of water for injection (WFI), bacteriostatic water for injection (BWFI), sodium chloride solutions (e.g., 0.9% (w/v) NaCl), glucose solution (e.g., 5% glucose), surfactant-containing solution (e.g., 0.01% polysorbate 20), pH buffer solution (e.g., phosphate buffer solution), Ringer's solution, and any combination thereof.

As used herein, the term "prevention" refers to a method performed to prevent or delay the occurrence of a disease or disorder or symptom in a subject. As used herein, the term "treatment" refers to a method performed to obtain a beneficial or desired clinical result. For the purposes of the present invention, beneficial or desired clinical results include (but are not limited to) alleviation of symptoms, reduction of the scope of disease, stabilization (i.e., no longer worsening) of the state of disease, delay or slowing of the progression of disease, improvement or alleviation of the state of disease, and relief of symptoms (whether partial or complete), whether detectable or undetectable. In addition, "treatment" may also refer to prolonging survival as compared to the expected survival if not receiving treatment.

As used herein, the term "subject" refers to a mammal, such as a human, a cynomolgus monkey, or a mouse.

As used herein, the term "effective amount" refers to an amount sufficient to obtain or at least partially obtain the desired effect. For example, an effective amount for preventing a disease refers to an amount sufficient to prevent, arrest, or delay the occurrence of the disease; an effective amount for treating a disease refers to an amount sufficient to cure or at least partially prevent the disease and its complications in a patient already suffering from the disease. Determining such an effective amount is well within the capabilities of those skilled in the art. For example, an effective amount for therapeutic use will depend on the severity of the disease to be treated, the overall state of the patient's own immune system, the patient's general condition such as age, weight and gender, the mode of administration, and other treatments administered simultaneously, etc.

### Beneficial effects of the invention

The present invention provides an antibody or antigen-binding fragment thereof targeting CCR8, which has a high binding affinity and good specificity to CCR8. Further, the heavy chain constant region is modified and a fully human antibody is obtained, which can specifically eliminate CCR8-positive cells through enhanced ADCC and ADCP activity while avoiding killing of CCR8-negative cells. Therefore, the antibody of the present invention can be used for a variety of purposes, including but not limited to enhancing immune response, inhibiting tumor growth, anti-infection and detecting CCR8 protein. In addition, the fully human antibody of the present invention can be safely administered to human subjects without inducing immunogenic reactions. Therefore, the antibody of the present invention has great clinical value.

The embodiments of the present invention will be described in detail below in conjunction with the drawings and examples, but those skilled in the art will understand that the following drawings and examples are only used to illustrate the present invention, rather than to limit the scope of the present invention. According to the following detailed description of the drawings and preferred embodiments, the various objects and advantages of the present invention will become apparent to those skilled in the art.

### Brief Description of the Drawings

Fig. 1 shows the binding activity of the anti-CCR8 antibody of the present application to human CCR8-overexpressing CHO cells (panel A in Fig. 1) and CHO cells (panel B in Fig. 1).
Fig. 2 shows the ADCC activity of the anti-CCR8 antibody of the present application based on CHO-huCCR8/Jurkat-CD16a-NFAT luciferase reporter gene cells (panel A in Fig. 2) and CHO/Jurkat-CD16a-NFAT luciferase reporter gene cells (panel B in Fig. 2).
Fig. 3 shows the ADCP activity of the anti-CCR8 antibody of the present invention based on CHO-huCCR8/Jurkat-CD32a-NFAT luciferase reporter gene cells (panel A in Fig. 3) and CHO/Jurkat-CD32a-NFAT luciferase reporter gene cells (panel B in Fig. 3).
Fig. 4 shows the cell killing activity of the anti-CCR8 antibody of the present invention based on CHO-huCCR8/PBMC.
Fig. 5 shows the anti-tumor effect of the anti-CCR8 antibody of the present invention in huCCR8 transgenic mouse CT-26 tumor model.
Fig. 6 shows the synergistic anti-tumor effect of the anti-mouse CCR8 antibody of the present invention combined with anti-PD-L1/TGF-βRII fusion protein in wild-type mouse CT-26 tumor model.

### Sequence information

The information of some sequences involved in the present invention is provided in Table 1 below.

**Table 1: Description of sequences**

| SEQ ID NO: | Description | Sequence |
|---|---|---|
| 1 | ADI-68741 VH CDR1 | GYTFTSYY |
| 2 | ADI-68741 VH CDR2 | INPGTGST |
| 3 | ADI-68741 VH CDR3 | ARDGAFRHKYFDL |
| 4 | ADI-68741 VH | QVQLVQSGAEVKKPGASVKVSCKASGYTFTSYYMHWVRQAPG QGLEWMGIINPGTGSTSHAQKFQGRVTMTRDTSTSTVYMELSSL RSEDTAVYYCARDGAFRHKYFDLWGRGTLVTVSS |
| 5 | ADI-68741 VL CDR1 | QSVSSY |
| 6 | ADI-68741 VL CDR2 | DAS |
| 7 | ADI-68741 VL CDR3 | QQYSAWPLT |
| 8 | ADI-68741 VL | EIVLTQSPATLSLSPGERATLSCRASQSVSSYLAWYQQKPGQA PRLLIYDASNRATGIPARFSGSGSGTDFTLTISSLEPEDFAVYY CQQYSAWPLTFGGGTKVEIK |
| 9 | Amino acid sequence of wild-type heavy chain constant region of human IgG1 | ASTKGPSVFPLAPSSKSTSGGTAALGCLVKDYFPEPVTVSWNS GALTSGVHTFPAVLQSSGLYSLSSVVTVPSSSLGTQTYICNVN HKPSNTKVDKKVEPKSCDKTHTCPPCPAPELLGGPSVFLFPPK PKDTLMISRTPEVTCVVVDVSHEDPEVKFNWYVDGVEVHNA KTKPREEQYNSTYRVVSVLTVLHQDWLNGKEYKCKVSNKAL PAPIEKTISKAKGQPREPQVYTLPPSRDELTKNQVSLTCLVKG FYPSDIAVEWESNGQPENNYKTTPPVLDSDGSFFLYSKLTVDK SRWQQGNVFSCSVMHEALHNHYTQKSLSLSPG |
| 10 | Amino acid sequence of mutated heavy chain constant region of human IgG1-GA | ASTKGPSVFPLAPSSKSTSGGTAALGCLVKDYFPEPVTVSWNS GALTSGVHTFPAVLQSSGLYSLSSVVTVPSSSLGTQTYICNVN HKPSNTKVDKKVEPKSCDKTHTCPPCPAPELLAGPSVFLFPPK PKDTLMISRTPEVTCVVVDVSHEDPEVKFNWYVDGVEVHNA KTKPREEQYNSTYRVVSVLTVLHQDWLNGKEYKCKVSNKAL PAPIEKTISKAKGQPREPQVYTLPPSRDELTKNQVSLTCLVKG FYPSDIAVEWESNGQPENNYKTTPPVLDSDGSFFLYSKLTVDK SRWQQGNVFSCSVMHEALHNHYTQKSLSLSPG |
| 11 | Amino acid sequence of kappa light chain constant region of human immunoglobulin | |
| 12 | Amino acid sequence of human CCR8 | MDYTLDLSVTTVTDYYYPDIFSSPCDAELIQTNGKLLLAVFYC LLFVFSLLGNSLVILVLVVCKKLRSITDVYLLNLALSDLLFVFS FPFQTYYLLDQWVFGTVMCKVVSGFYYIGFYSSMFFITLMSV DRYLAVVHAVYALKVRTIRMGTTLCLAVWLTAIMATIPLLVF YQVASEDGVLQCYSFYNQQTLKWKIFTNFKMNILGLLIPFTIF MFCYIKILHQLKRCQNHNKTKAIRLVLIVVIASLLFWVPFNVV LFLTSLHSMHILDGCSISQQLTYATHVTEIISFTHCCVNPVIYAF VGEKFKKHLSEIFQKSCSQIFNYLGRQMPRESCEKSSSCQQHS SRSSSVDYIL |
| 13 | Amino acid sequence of heavy chain constant region of mouse IgG2a | AKTTAPSVYPLAPVCGDTTGSSVTLGCLVKGYFPEPVTLTWN SGSLSSGVHTFPAVLQSDLYTLSSSVTVTSSTWPSQSITCNVA HPASSTKVDKKIEPRGPTIKPCPPCKCPAPNLLGGPSVFIFPPKI KDVLMISLSPIVTCVVVDVSEDDPDVQISWFVNNVEVHTAQT QTHREDYNSTLRVVSALPIQHQDWMSGKEFKCKVNNKDLPA PIERTISKPKGSVRAPQVYVLPPPEEEMTKKQVTLTCMVTDFM PEDIYVEWTNNGKTELNYKNTEPVLDSDGSYFMYSKLRVEK KNWVERNSYSCSVVHEGLHNHHTTKSFSRTPGK |
| 14 | Amino acid sequence of kappa light chain constant region of mouse immunoglobulin | RADAAPTVSIFPPSSEQLTSGGASVVCFLNNFYPKDINVKWKI DGSERQNGVLNSWTDQDSKDSTYSMSSTLTLTKDEYERHNS YTCEATHKTSTSPIVKSFNRGEC |
| 15 | Amino acid sequence of heavy chain of anti-PD-L1/TGF-βRII fusion protein | EVQLVESGGGLVQPGGSLRLSCAASGFTFSDSWIHWVRQAPG KGLEWVAWISPYGGSTYYADSVKGRFTISADTSKNTAYLQM NSLRAEDTAVYYCARRHWPGGFDYWGQGTLVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNT KVDKKVEPKSCDKTHTCPPCPAPELLGGPSVFLFPPKPKDTLM ISRTPEVTCVVVDVSHEDPEVKFNWYVDGVEVHNAKTKPRE |
| | | EQYASTYRVVSVLTVLHQDWLNGKEYKCKVSNKALPAPIEK TISKAKGQPREPQVYTLPPSREEMTKNQVSLTCLVKGFYPSDI AVEWESNGQPENNYKTTPPVLDSDGSFFLYSKLTVDKSRWQ QGNVFSCSVMHEALHNHYTQKSLSLSPGKGGGGSGGGGSGG GGSGGGGSGIPPHVQKSVNNDMIVTDNNGAVKFPQLCKFCD VRFSTCDNQKSCMSNCSITSICEKPQEVCVAVWRKNDENITLE TVCHDPKLPYHDFILEDAASPKCIMKEKKKPGETFFMCSCSSD ECNDNIIFSEEYNTSNPD |
| 16 | Amino acid sequence of light chain of anti-PD-L1/TGF-βRII fusion protein | DIQMTQSPSSLSASVGDRVTITCRASQDVSTAVAWYQQKPGK APKLLIYSASFLYSGVPSRFSGSGSGTDFTLTISSLQPEDFATY YCQQYLYHPATFGQGTKVEIKRTVAAPSVFIFPPSDEQLKSGT ASVVCLLNNFYPREAKVQWKVDNALQSGNSQESVTEQDSKD STYSLSSTLTLSKADYEKHKVYACEVTHQGLSSPVTKSFNRG EC |

### Specific Models for Carrying Out the present invention

The present invention is now described with reference to the following examples which are intended to illustrate the present invention (but not to limit the present invention).

Unless otherwise specified, the experiments and methods described in the examples were basically carried out according to conventional methods well known in the art and described in various references. For example, the conventional techniques of immunology, biochemistry, chemistry, molecular biology, microbiology, cell biology, genomics and recombinant DNA used in the present invention could be found in Sambrook, Fritsch and Maniatis, MOLECULAR CLONING: A LABORATORY MANUAL, 2nd edition (1989); CURRENT PROTOCOLS IN MOLECULAR BIOLOGY (F. M. Ausubel et al., eds., (1987)); METHODS IN ENZYMOLOGY series (Academic Publishing Company): PCR 2: A PRACTICAL METHOD. APPROACH) (M. J. MacPherson, B.D. Hames and G.R. Taylor, ed. (1995)), and ANIMAL CELL CULTURE (R. I. Freshney, ed. (1987)).

In addition, if the specific conditions were not specified in the examples, they were carried out according to conventional conditions or conditions recommended by the manufacturer. The reagents or instruments used without indicating the manufacturer were all conventional products that could be obtained commercially. It is known to those skilled in the art that the examples describe the present invention by way of example and are not intended to limit the scope sought to be protected of by the present invention. All the public cases and other references mentioned herein are incorporated herein by reference in their entirety.

### Example 1. Preparation of antibodies

### 1.1 Screening of fully human anti-CCR8 antibodies using yeast display technology

Based on the yeast antibody display library (Adimab, see WO2009036379, WO2010105256 and WO2012009568), multiple methods such as binding and enrichment of human CCR8-overexpressing cells were used to screen and enrich yeast cells capable of specifically binding to human CCR8. The yeast cells obtained by screening were shaken and induced at 30°C for 48 hours to secrete and express the target anti-CCR8 antibodies (full-length IgG). After the induction, the yeast cells were removed by centrifugation at 1300rpm for 10 minutes, and the supernatant was harvested. The anti-CCR8 antibodies in the supernatant were purified using Protein A, eluted with pH2.0 acetic acid solution, and the anti-CCR8 antibodies were harvested.

### 1.2 Affinity maturation of antibodies

In order to obtain anti-human CCR8 antibodies with higher affinity, the above-screened antibodies were optimized by, but not limited to, the following methods: CDRH1/CDRH2 mutation screening; VHmut mutation screening; CDRL1/CDRL2/CDRL3 mutation screening, and the like. The specific CDR1 to CDR3 sequences (encoded by the IMGT coding system) and VH and VL sequences of the screened antibodies were shown in Table 1.

### 1.3 Expression and purification of antibodies

The heavy chain variable region of candidate clone ADI-68741 (with amino acid sequence as set forth in SEQ ID NO: 4) was fused to the human IgG1 wild-type heavy chain constant region (with amino acid sequence as set forth in SEQ ID NO: 9), the human IgG1-GA modified heavy chain constant region (G1-GA) (with amino acid sequence as set forth in SEQ ID NO: 10), and the mouse IgG2a heavy chain constant region (with amino acid sequence as set forth in SEQ ID NO: 13), respectively, and cloned into the pcDNA3.1 vector; and its light chain variable region (with amino acid sequence as set forth in SEQ ID NO: 8) was linked to the human immunoglobulin kappa light chain constant region (with amino acid sequence as set forth in SEQ ID NO: 11) and the mouse immunoglobulin kappa light chain constant region (with amino acid sequence as set forth in SEQ ID NO: 14), respectively, and cloned into the pcDNA3.1 vector.

Transient expression and purification via CHO cell or fucose knockout CHO cell expression system, and the specific operation was as follows: Chemical transfection method was used to transfer the pcDNA3.1 vector with antibody heavy chain and light chain into CHO cells or fucose knockout CHO cells, then the cells were cultured at 37°C, 8% CO₂ for 7 days. The cell fluid was collected and centrifuged at 13000rpm for 20 minutes. The supernatant was collected and purified with Protein A, the antibody purity was detected by SEC, and the endotoxin content was controlled at the same time. Five antibodies were finally obtained, named ADI-68741-G1 (the variable regions of the candidate clone were constructed to the constant region of human IgG1 wild-type heavy chain and human immunoglobulin kappa light chain), ADI-68741-GA (the variable regions of candidate clone were constructed to the constant region of human IgG1-GA modified heavy chain and human immunoglobulin kappa light chain), ADI-68741-G1 (afucosylated) (the construction method was the same as ADI-68741-G1, the difference being that a fucose knockout CHO cell expression system was used), ADI-68741-GA (afucosylated) (the construction method was the same as ADI-68741-GA, the difference being that a fucose knockout CHO cell expression system was used), ADI-68741-mG2a (the variable regions of the candidate clone were constructed to the constant region of mouse IgG2a heavy chain and mouse immunoglobulin kappa light chain).

### Example 2. Binding activity of anti-CCR8 antibodies to human CCR8-overexpressing CHO cells or empty CHO cells

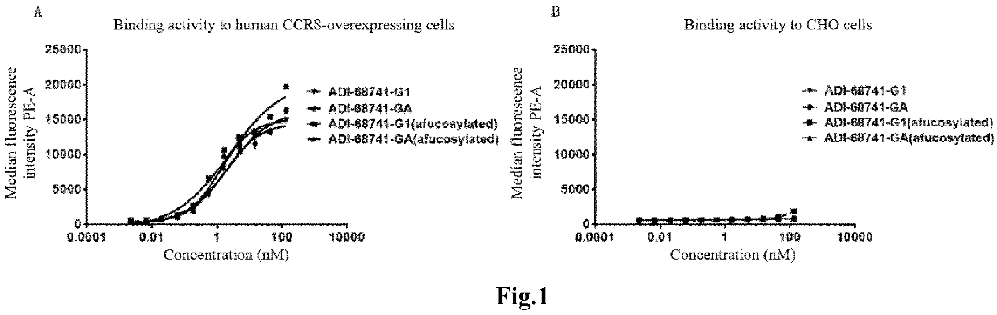

Specifically, human CCR8-overexpressing CHO cells (named CHO-huCCR8 cells) were generated via pressure screening by transfecting the pCHO1.0 vector (purchased from Invitrogen) cloned into the human CCR8 (with amino acid sequence as set forth in SEQ ID NO: 12) cDNA of MCS. The expanded cultured CHO-huCCR8 cells or empty CHO cells were adjusted to an appropriate cell density and added to a 96-well flow plate. After centrifugation, the gradiently diluted samples to be tested were added and incubated at 4°C for 30 minutes, washed twice with PBS, added with fluorescent secondary antibody correspondingly diluted to appropriate concentration, incubated at 4°C for 30 minutes, and washed twice with PBS. The cells were resuspended by adding PBS, and detected on a CytoFlex flow cytometer, and the corresponding MFI was calculated. Graphpad software was used to generate graphs, and the results were shown in Fig. 1. The anti-CCR8 antibodies ADI-68741-G1, ADI-68741-GA, ADI-68741-G1 (afucosylated), and ADI-68741-GA (afucosylated) of the present invention all could specifically bind to the CCR8 expressed on CHO cells, and their EC₅₀ values for binding to CHO-huCCR8 cells were 1.65nM, 1.21nM, 2.41nM, and 2.30nM, respectively, while they had no binding activity to empty CHO cells.

### Example 3. Detection of in vitro ADCC activity of anti-CCR8 antibodies

Based on the luciferase reporter gene system, the in vitro ADCC activity of the anti-CCR8 antibodies of the present invention was detected.

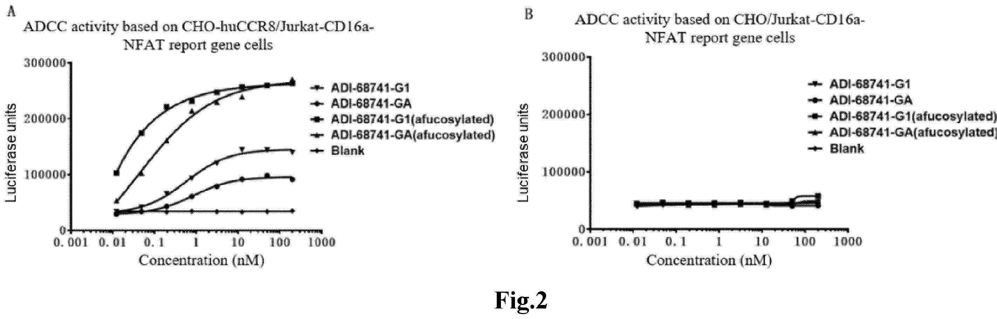

Specifically, Jurkat-CD16a-NFAT-Luciferase-ADCC effector cells (purchased from Promega) were expanded and resuspended to 4×10⁶ cells/mL in 1640 culture medium containing 10% low IgG FBS. The CHO-huCCR8 cells and empty CHO cells were resuspended in 1640 medium containing 10% low IgG FBS and diluted to 0.8×10⁶ cells/mL. The above two cell suspensions were separately mixed well with Jurkat-CD16a-NFAT-Luciferase-ADCC effector cells at a ratio of 1:1, added to a sterile 96-well white bottom plate at 50 µL/well, then added with the antibody sample to be tested gradiently diluted in 1640 medium containing 10% low IgG FBS, and incubated for 6 hours at 37°C and 5% CO₂. After the culture was completed, the cells were taken out, equilibrated at room temperature for 5 minutes, added with Bio-Glo TM reagent at 100 µL/well, and the luminescence signal value was read using a multifunctional microplate reader. The results were shown in Fig. 2. The results showed that the anti-CCR8 antibodies of the present invention exhibited ADCC activity only in the CHO-huCCR8 cells, and the afucosylated antibodies ADI-68741-G1 (afucosylated) (EC₅₀ < 0.01nM) and ADI-68741-GA (afucosylated) (EC₅₀ = 0.04nM) showed stronger ADCC activity than ADI-68741-G1 (EC₅₀ = 0.57nM) and ADI-68741-GA (EC₅₀ = 0.85nM).

### Example 4. Detection of in vitro ADCP activity of anti-CCR8 antibodies

Based on the luciferase reporter gene system, the in vitro ADCP activity of the anti-CCR8 antibodies of the present invention was detected.

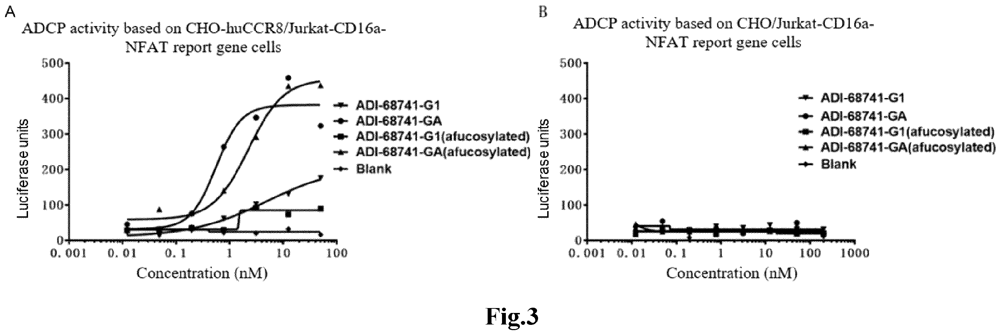

Specifically, Jurkat-CD32a-NFAT-Luciferase-ADCP effector cells (purchased from Rhino Bio) were expanded and resuspended in 1640 medium to 4×10⁶ cells/mL. CHO-huCCR8 cells and empty CHO cells were resuspended in 1640 medium and diluted to 3.2×10⁶ cells/mL. The above two cell suspensions were separately mixed well with Jurkat-CD32a-NFAT-Luciferase-ADCP effector cells in a ratio of 1:1, added to a sterile 96-well white bottom plate at 50 µL/well, then added with the antibody sample to be tested gradiently diluted in 1640 medium, and incubated at 37°C and 5% CO₂ for 6 hours. After the culture was completed, the cells were taken out, equilibrated at room temperature for 5 minutes, and added with Bio-Glo TM reagent at 100 µL/well, and the luminescence signal value was read using a multifunctional microplate reader. The results were shown in Fig. 3. The results showed that the anti-CCR8 antibodies of the present invention exhibited ADCP activity only in the CHO-huCCR8 cells, and the antibodies ADI-68741-GA (EC₅₀=0.56nM) and ADI-68741-GA (afucosylated) (EC₅₀=2.24nM) with GA-engineered at the Fc-terminal showed stronger ADCP activity than ADI-68741-G1 (EC₅₀=4.90nM) and ADI-68741-G1 (afucosylated) (Not fit).

### Example 5. Detection of anti-CCR8 antibodies in PBMC cell-mediated in vitro killing activity

Based on the PBMC killing system, the in vitro killing activity of the anti-CCR8 antibodies of the present invention was detected.

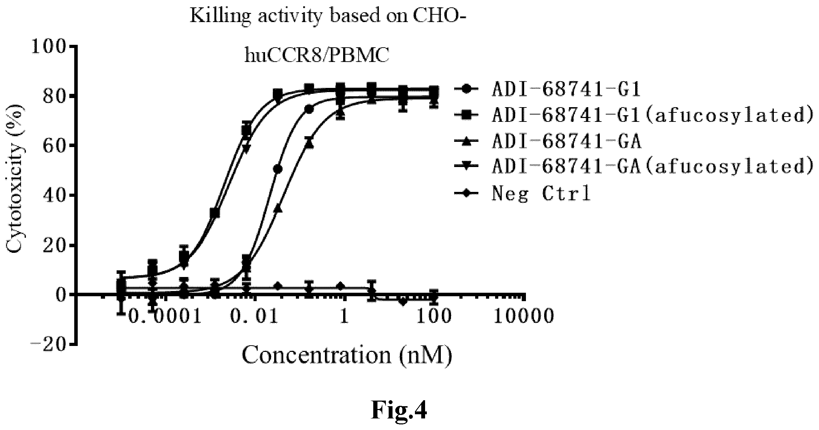

Specifically, human PBMC cells were resuscitated, cultured overnight to adhere to the wall to remove monocytes, and the PBMC cells were collected on the second day and added to a black 96-well cell culture plate with clear bottom at 1×10⁵ cells/well. The CHOS-huCCR8 target cells were stained using the CellTrace Violet kit, adjusted to reach a cell density of 1×10⁴ cells/well, and inoculated into the above black 96-well clear bottom cell culture plate. Then, the gradiently diluted antibodies to be tested were added to the cell wells and incubated for 48 hours. After 48 hours, DAPI fluorescence signals were collected using the Cytation 5 and the corresponding killing activity was calculated. The results were shown in Fig. 4. The results showed that the anti-CCR8 antibodies of the present invention exhibited in vitro PBMC cell-mediated ADCC killing activity, and the afucosylated antibodies ADI-68741-G1 (afucosylated) (EC₅₀=0.0021nM) and ADI-68741-GA (afucosylated) (EC₅₀=0.0027nM) exhibited stronger PBMC killing activity than ADI-68741-G1 (EC₅₀=0.0213nM) and ADI-68741-GA (EC₅₀=0.0433nM).

### Example 6. In vivo pharmacodynamic study of anti-CCR8 antibody in huCCR8 KI mice

In this experiment, huCCR8 KI mice (purchased from Shanghai Model Organisms Center, Inc.) were in vivo inoculated with CT-26 colon cancer cells (purchased from Jiangsu GemPharmatech Co., Ltd.) to determine the anti-tumor effect of the anti-CCR8 antibody of the present invention.

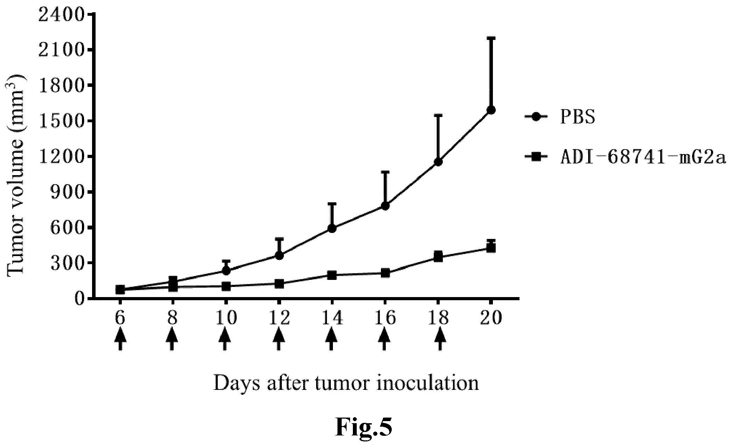

Specifically, a CT-26 cell tumor-bearing mouse model was first established by subcutaneous inoculation. When the average tumor volume reached 80 mm³, the mice were divided into groups. The ADI-68741-mG2a antibody of the present invention was injected intraperitoneally for treatment (10 mg/Kg). The changes in tumor volume and body weight of each group of mice were monitored. The monitoring frequency was once every 2 to 3 days, and the monitoring was continued for 2 to 3 weeks. The results were shown in Fig. 5. The results showed that the anti-CCR8 antibody ADI-68741-mG2a of the present invention could significantly inhibit the growth of mouse tumors.

### Example 7. Synergistic pharmacodynamic study of anti-CCR8 antibody and anti-PD-L1/TGF-βRII fusion protein in wild-type Balb/c mice

In order to explore the potential combination of CCR8 antibody for future clinical applications, CT-26 tumor cells inoculated into wild-type Balb/c mice were used in the present invention to determine the synergistic anti-tumor effect of anti-CCR8 antibody and anti-PD-L1/TGF-βRII fusion protein (with heavy chain amino acid sequence as set forth in SEQ ID NO: 15, and light chain amino acid sequence as set forth in SEQ ID NO: 16).

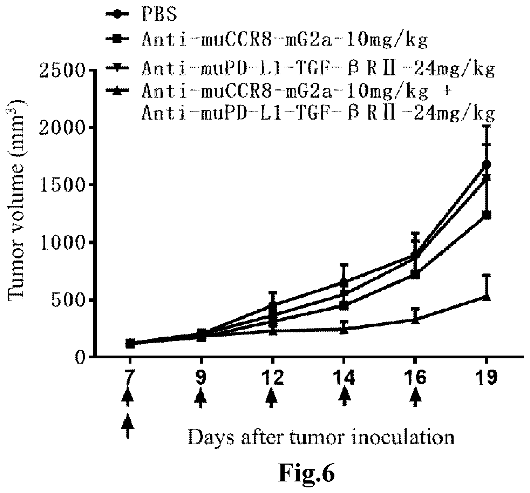

Specifically, a CT-26 cell tumor-bearing mouse model was first established by subcutaneous inoculation. When the average tumor volume reached 150 mm³, the mice were divided into groups, and treated with anti-CCR8 antibody and/or anti-PD-L1/TGF-βRII fusion protein by intraperitoneal injection. The changes in tumor volume and body weight of mice in each group were monitored. The monitoring frequency was once every 2-3 days, and the monitoring was continued for 2 to 3 weeks. The dosage and route of administration were shown in Table 2. The results were shown in Fig. 6. The results showed that the tumor inhibitory activity of the anti-CCR8 antibody combined with anti-PD-L1/TGF-βRII fusion protein group was significantly better than that of the corresponding two single-drug treatment groups. This suggested that CCR8 antibodies in combination with anti-PD-L1/TGF-βRII fusion proteins may have potential for future clinical development.

**Table 2: Dosage regimen of anti-CCR8 antibody and anti-PD-L1/TGF-βRII fusion protein**

| Group | Dosage | Times of administration |
|---|---|---|
| PBS | N/A | 5 |
| Anti-muCCR8-mG2a | 10 mg/kg | 5 |
| Anti-muPD-L1/GF-βRII fusion protein | 24 mg/kg | Single administration |
| Anti-muCCR8-mG2a + Anti-muPD-L1/GF-βRII fusion protein | 10 mg/kg + 24 mg/kg | 5 + single administration |

Although the specific embodiments of the present invention have been described in detail, those skilled in the art will understand that various modifications and changes can be made to the details based on all the teachings published, and these changes are within the scope of protection of the present invention. All divisions of the present invention are given by the attached claims and any equivalents thereof.

## Claims

1. An antibody or antigen-binding fragment thereof capable of specifically binding to CCR8, wherein the antibody or antigen-binding fragment thereof comprises:
(a) a heavy chain variable region (VH) comprising the following 3 complementarity determining regions (CDRs):
(i) a VH CDR1, which consists of the following sequence: SEQ ID NO: 1, or a sequence having a substitution, deletion or addition of one or several amino acids (e.g., a substitution, deletion or addition of 1, 2 or 3 amino acids) as compared thereto,
(ii) a VH CDR2, which consists of the following sequence: SEQ ID NO: 2, or a sequence having a substitution, deletion or addition of one or several amino acids (e.g., a substitution, deletion or addition of 1, 2 or 3 amino acids) as compared thereto, and
(iii) a VH CDR3, which consists of the following sequence: SEQ ID NO: 3, or a sequence having a substitution, deletion or addition of one or several amino acids (e.g., a substitution, deletion or addition of 1, 2 or 3 amino acids) as compared thereto;
and/or,
(b) a light chain variable region (VL) comprising the following 3 complementarity determining regions (CDRs):
(iv) a VL CDR1, which consists of the following sequence: SEQ ID NO: 5, or a sequence having a substitution, deletion or addition of one or several amino acids (e.g., a substitution, deletion or addition of 1, 2 or 3 amino acids) as compared thereto,
(v) a VL CDR2, which consists of the following sequence: SEQ ID NO: 6, or a sequence having a substitution, deletion or addition of one or several amino acids (e.g., a substitution, deletion or addition of 1, 2 or 3 amino acids) as compared thereto, and
(vi) a VL CDR3, which consists of the following sequence: SEQ ID NO: 7, or a sequence having a substitution, deletion or addition of one or several amino acids (e.g., a substitution, deletion or addition of 1, 2 or 3 amino acids) as compared thereto;
preferably, the substitution as described in any one of (i) to (vi) is a conservative substitution;
preferably, the CDRs as described in any one of (i) to (vi) are defined according to the Kabat, IMGT or Chothia numbering system;
preferably, the CDRs as described in any one of (i) to (vi) are defined according to the IMGT numbering system;
preferably, the antibody or antigen-binding fragment thereof comprises: the following 3 heavy chain CDRs: a VH CDR1 as set forth in SEQ ID NO: 1, a VH CDR2 as set forth in SEQ ID NO: 2, a VH CDR3 as set forth in SEQ ID NO: 3; and/or, the following 3 light chain CDRs: a VL CDR1 as set forth in SEQ ID NO: 5, a VL CDR2 as set forth in SEQ ID NO: 6, a VL CDR3 as set forth in SEQ ID NO: 7.

2. The antibody or antigen-binding fragment thereof according to claim 1, wherein the antibody or antigen-binding fragment thereof comprises:
(a) a heavy chain variable region (VH), which comprises an amino acid sequence selected from the group consisting of:
(i) a sequence as set forth in SEQ ID NO: 4;
(ii) a sequence having a substitution, deletion or addition of one or several amino acids (e.g., a substitution, deletion or addition of 1, 2, 3, 4 or 5 amino acids) as compared to the sequence as set forth in SEQ ID NO: 4; and
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence as set forth in SEQ ID NO: 4;
and/or
(b) a light chain variable region (VL), which comprises an amino acid sequence selected from the group consisting of:
(iv) a sequence as set forth in SEQ ID NO: 8;
(v) a sequence having a substitution, deletion or addition of one or several amino acids (e.g., a substitution, deletion or addition of 1, 2, 3, 4 or 5 amino acids) as compared to the sequence as set forth in SEQ ID NO: 8; and
(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% with the sequence shown in SEQ ID NO: 8;
preferably, the substitution as described in (ii) or (v) is a conservative substitution;
preferably, the antibody or antigen-binding fragment thereof comprises: a VH having a sequence as set forth in SEQ ID NO: 4 and a VL having a sequence as set forth in SEQ ID NO: 8.

3. The antibody or antigen-binding fragment thereof according to claim 1 or 2, wherein the antibody or antigen-binding fragment thereof comprises a constant region derived from a mammalian immunoglobulin or a variant thereof;
preferably, the antibody or antigen-binding fragment thereof comprises:
(a) a heavy chain constant region (CH) of a mammalian immunoglobulin or a variant thereof, wherein the variant has a substitution, deletion or addition of one or several amino acids or any combination thereof (e.g., a substitution, deletion or addition of up to 20, up to 15, up to 10, or up to 5 amino acids or any combination thereof; e.g., a substitution, deletion or addition of 1, 2, 3, 4 or 5 amino acids or any combination thereof) as compared to the sequence from which it is derived; and/or
(b) a light chain constant region (CL) of a mammalian immunoglobulin or a variant thereof, wherein the variant has a substitution, deletion or addition of one or several amino acids or any combination thereof (e.g., a substitution, deletion or addition of up to 20, up to 15, up to 10, or up to 5 amino acids or any combination thereof; e.g., a substitution, deletion or addition of 1, 2, 3, 4 or 5 amino acids or any combination thereof) as compared to the sequence from which it is derived;
preferably, the mammal is selected from a mouse or a human.

4. The antibody or antigen-binding fragment thereof according to claim 3, wherein the heavy chain constant region is selected from IgG, IgM, IgE, IgD or IgA;
preferably, the heavy chain constant region is a human or mouse IgG heavy chain constant region, for example, a human IgG1, IgG2, IgG3 or IgG4 heavy chain constant region; for example, a mouse IgG1, IgG2a, IgG2b, IgG2c or IgG3 heavy chain constant region;
preferably, the heavy chain constant region is selected from the group consisting of a human IgG1 heavy chain constant region and a mouse IgG2a heavy chain constant region;
preferably, the variant is a variant of a human IgG1 heavy chain constant region;
preferably, the variant is mutated to alanine at the position corresponding to position 119 of the human IgG1 heavy chain constant region;
preferably, the antibody or antigen-binding fragment thereof is hypofucosylated or afucosylated;
preferably, the antibody or antigen-binding fragment thereof comprises a heavy chain constant region (CH) as set forth in SEQ ID NO: 9, 10 or 13;
preferably, the light chain constant region is a κ light chain constant region or a λ light chain constant region;
preferably, the light chain constant region is a mouse or human κ light chain constant region;
preferably, the antibody or antigen-binding fragment thereof comprises a light chain constant region (CL) as set forth in SEQ **ID** NO: 11 or 14.

5. The antibody or antigen-binding fragment thereof according to any one of claims 1 to 4, wherein the antigen-binding fragment is selected from the group consisting of Fab, Fab', (Fab')², Fv, disulfide-linked Fv, scFv, diabody and single-domain antibody (sdAb); and/or, the antibody is a murine antibody, a chimeric antibody, a humanized antibody or a multispecific antibody.

6. An isolated nucleic acid molecule, which encodes the antibody or antigen-binding fragment thereof according to any one of claims 1 to 5.

7. A vector, which comprises the nucleic acid molecule according to claim 6; preferably, the vector is a cloning vector or an expression vector.

8. A host cell, which comprises the nucleic acid molecule according to claim 6 or the vector according to claim 7;
preferably, the host cell is a mammalian cell;
preferably, the host cell is a fucose knockout or non-knockout cell.

9. A method for preparing the antibody or antigen-binding fragment thereof according to any one of claims 1 to 5, comprising culturing the host cell according to claim 8 under a condition that allows expression of the antibody or antigen-binding fragment thereof, and recovering the antibody or antigen-binding fragment thereof from a culture of the cultured host cell.

10. An immunoconjugate, which comprises the antibody or antigen-binding fragment thereof according to any one of claims 1 to 5, and a therapeutic agent linked to the antibody or antigen-binding fragment thereof or a multispecific molecule;
preferably, the therapeutic agent is selected from a cytotoxic agent;
preferably, the therapeutic agent is selected from the group consisting of alkylating agent, mitotic inhibitor, antitumor antibiotic, antimetabolite, topoisomerase inhibitor, tyrosine kinase inhibitor, radionuclide agent, and any combination thereof;
preferably, the immunoconjugate is an antibody-drug conjugate (ADC).

11. A pharmaceutical composition, which comprises the antibody or antigen-binding fragment thereof according to any one of claims 1 to 5, or the immunoconjugate according to claim 10, and a pharmaceutically acceptable carrier and/or excipient;
preferably, the pharmaceutical composition further comprises an additional pharmaceutically active agent;
preferably, the additional pharmaceutically active agent is a drug with an anti-tumor activity, such as an alkylating agent, a mitotic inhibitor, an anti-tumor antibiotic, an antimetabolite, a topoisomerase inhibitor, a tyrosine kinase inhibitor, a radionuclide agent, a radiosensitizer, an anti-angiogenic agent, a cytokine, a molecular targeted drug, an immune checkpoint inhibitor or an oncolytic virus;
preferably, the antibody or antigen-binding fragment thereof or the immunoconjugate and the additional pharmaceutically active agent are provided as separate components or as components in the same composition;
preferably, the additional pharmaceutically active agent is an anti-PD-L1/TGF-βRII fusion protein.

12. A kit, which comprises the antibody or antigen-binding fragment thereof according to any one of claims 1 to 5;
preferably, the antibody or antigen-binding fragment thereof comprises a detectable label, such as an enzyme (e.g., horseradish peroxidase), a radionuclide, a fluorescent dye, a luminescent substance (e.g., a chemiluminescent substance) or biotin;
preferably, the kit further comprises a second antibody, which is capable of specifically recognizing the antibody or antigen-binding fragment thereof according to any one of claims 1 to 5;
preferably, the second antibody further comprises a detectable label, such as an enzyme (e.g., horseradish peroxidase), a radionuclide, a fluorescent dye, a luminescent substance (e.g., a chemiluminescent substance) or biotin.

13. A chimeric antigen receptor, which comprises an antigen-binding domain of the antibody or antigen-binding fragment thereof according to any one of claims 1 to 5;
preferably, the antigen-binding domain comprises a heavy chain variable region and a light chain variable region of the antibody or antigen-binding fragment thereof according to any one of claims 1 to 5;
preferably, the antigen-binding domain is a scFv;
preferably, the chimeric antigen receptor is expressed by an immune effector cell (e.g., a T cell).

14. A method for inhibiting growth of a tumor cell expressing CCR8 and/or killing the tumor cell, comprising: contacting the tumor cell with an effective amount of the antibody or antigen-binding fragment thereof according to any one of claims 1 to 5, or the immunoconjugate according to claim 10, or the pharmaceutical composition according to claim 11, or the chimeric antigen receptor according to claim 13.

15. Use of the antibody or antigen-binding fragment thereof according to any one of claims 1 to 5, or the immunoconjugate according to claim 10, or the pharmaceutical composition according to claim 11, or the chimeric antigen receptor according to claim 13, in the manufacture of a medicament for preventing and/or treating a tumor in a subject (e.g., a human);
preferably, the medicament further comprises an additional pharmaceutically active agent;
preferably, the additional pharmaceutically active agent is a drug with an anti-tumor activity, such as an alkylating agent, a mitotic inhibitor, an anti-tumor antibiotic, an antimetabolite, a topoisomerase inhibitor, a tyrosine kinase inhibitor, a radionuclide agent, a radiosensitizer, an anti-angiogenic agent, a cytokine, a molecular targeted drug, an immune checkpoint inhibitor or an oncolytic virus;
preferably, the additional pharmaceutically active agent is an anti-PD-L1/TGF-βRII fusion protein;
preferably, the tumor expresses CCR8;
preferably, the tumor involves a tumor cell expressing CCR8; preferably, the CCR8 is expressed on the surface of the tumor cell;
preferably, the tumor is selected from the group consisting of non-small cell lung cancer, small cell lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoids, Merkel cell carcinoma and other hematological malignancies, such as classical Hodgkin lymphoma (CHL), primary mediastinal large B-cell lymphoma, T-cell/histiocyte-rich B-cell lymphoma, EBV-positive and - negative PTLD and EBV-associated diffuse large B-cell lymphoma (DLBCL), plasmablastic lymphoma, extranodal NK/T-cell lymphoma, nasopharyngeal carcinoma and HHV8-associated primary effusion lymphoma, Hodgkin lymphoma, central nervous system (CNS) tumors, such as primary CNS lymphoma, spinal axis tumor, brainstem glioma;
preferably, the subject is a mammal, such as a human.

16. Use of the antibody or antigen-binding fragment thereof according to any one of claims 1 to 5 in the manufacture of a kit for determining whether a tumor can be treated by a CCR8-targeting anti-tumor therapy;
(1) contacting a sample containing a cell of the tumor with the antibody or antigen-binding fragment thereof according to any one of claims 1 to 5;
(2) detecting formation of a complex comprising the antibody or antigen-binding fragment thereof and CCR8;
preferably, the antibody or antigen-binding fragment thereof comprises a detectable label;
preferably, the CCR8 is a CCR8 of a mammal (e.g., human, mouse);
preferably, the tumor is selected from the group consisting of non-small cell lung cancer, small cell lung cancer, renal cell carcinoma, colorectal cancer, ovarian cancer, breast cancer, pancreatic cancer, gastric cancer, bladder cancer, esophageal cancer, mesothelioma, melanoma, head and neck cancer, thyroid cancer, sarcoma, prostate cancer, glioblastoma, cervical cancer, thymic cancer, leukemia, lymphoma, myeloma, mycosis fungoids, Merkel cell carcinoma and other hematological malignancies, such as classical Hodgkin lymphoma (CHL), primary mediastinal large B-cell lymphoma, T-cell/histiocyte-rich B-cell lymphoma, EBV-positive and - negative PTLD and EBV-associated diffuse large B-cell lymphoma (DLBCL), plasmablastic lymphoma, extranodal NK/T-cell lymphoma, nasopharyngeal carcinoma and HHV8-related primary effusion lymphoma, Hodgkin lymphoma, central nervous system (CNS) tumors, such as primary CNS lymphoma, spinal axis tumor, brainstem glioma.
